# EUROPÄISCHE PATENTSCHRIFT

(11) **EP 0 788 331 B1**
(45) Veröffentlichungstag und Bekanntmachung des Hinweises auf die Patenterteilung: **24.05.2000**
(21) Anmeldenummer: 95935357.4
(22) Anmeldetag: 19.10.1995
(51) Int. Cl.: A61B 17/60

(54) **KLEMMKUPPLUNG ZUR FESTLEGUNG VON KNOCHENSCHRAUBEN**
CLAMP COUPLING FOR SECURING BONE SCREWS
SYSTEME D'ACCOUPLEMENT A SERRAGE DESTINE A LA FIXATION DE VIS POUR FRACTURE OSSEUSE

(30) Priorität: 24.10.1994 US 327915
(43) Veröffentlichungstag der Anmeldung: 13.08.1997
(73) Patentinhaber: Pennig, Dietmar, Dr. med., 50935 Köln (DE)
(72) Erfinder: Pennig, Dietmar, Dr. med., 50935 Köln (DE)
(74) Vertreter: Habbel, Hans-Georg, Dipl.-Ing.
(86) Internationale Anmeldenummer: DE9501475
(87) Internationale Veröffentlichungsnummer: WO9612443

(56) Entgegenhaltungen:
- EP-A- 0 522 355
- WO-A-93/08758
- DE-A- 4 139 700

## Beschreibung

Diese Erfindung bezieht sich auf Osteosynthesehilfsmittel und insbesondere auf eine Verbindung für einen Fixateur.

Das US-Patent 43 12 336 beschreibt einen äußeren Fixateur, der aus einem Mittelteil und Befestigungsmitteln an jedem der Enden des Mittelteiles besteht. Jedes der Befestigungsmittel ist dazu ausgebildet, erstens Knochenschrauben oder Pins aufzunehmen und am Ort festzulegen und zweitens eine Kugelgelenkverbindung zu dem Mittelteil zu erreichen über einen Bajonettanschluß oder Gewindeanschluß. In dieser Art sind die Knochenschrauben mit dem Mittelteil des Fixateurs verbunden. Die Kugelgelenkverbindungen, die an den Enden angeordnet sind, können im Hinblick auf ihre Winkelstellung eingestellt werden bis zu einem Winkel von ca. 40 bis 45°, d. h. + - 20° oder mehr im Hinblick auf die Achse des Mittelteiles des Fixateurs, dessen Winkel in manchen Fällen unzureichend ist.

Eine gattungsbildende Einrichtung wird in der EP 522 355 A1 beschrieben. Um die bekannte Klemmkupplung dahingehend zu verbessern, daß ohne großen Aufwand auch größere Abwinkelungen zwischen dem zentralen Teil des Fixateurs und der eigentlichen Klemmkupplung möglich sind, wird ein Drehgelenk zwischen dem Kugelgelenkanschluß und der Halbschale eingeschaltet, die den eigentlichen Kugelgelenkanschluß trägt.

Ziel der vorliegenden Erfindung ist, die gattungsbildende Einrichtung derart zu verbessern, daß eine größere Universalität der Winkeleinstellung des Drehgelenkes erreichbar ist.

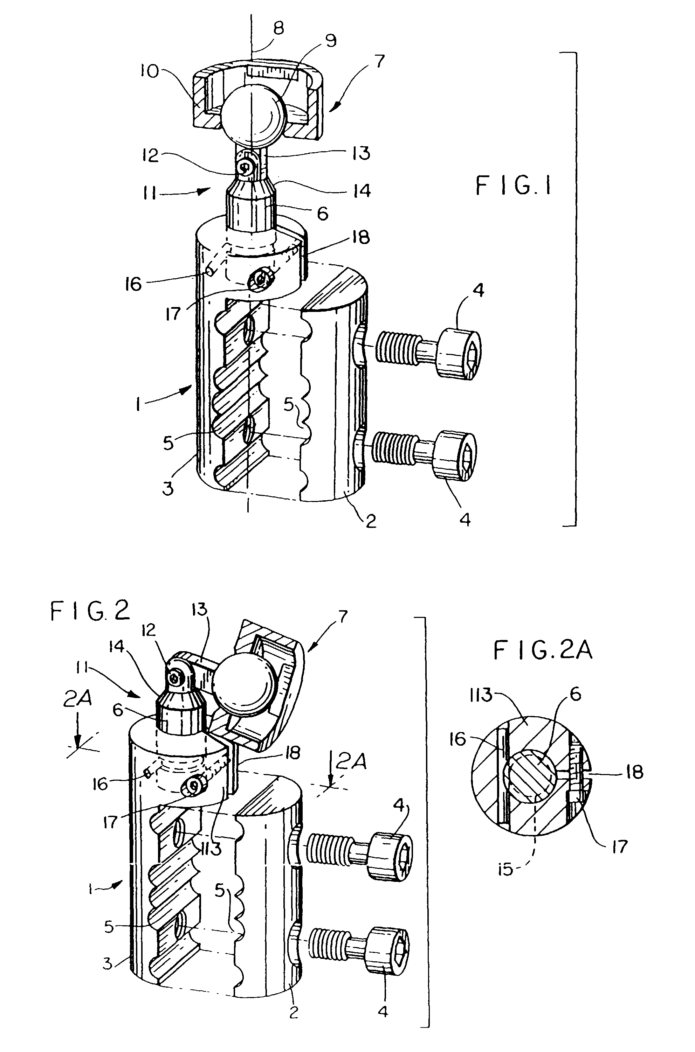

In einer ersten Ausführungsform der Erfindung wird dieses Ziel dadurch erreicht, daß eine Befestigungsverbindung vorgesehen ist, bei der ein Drehgelenk zwischen der Kugelgelenkverbindung des Fixateurkörpers und der Klemmhalbschale angeordnet ist, die das Kugelverbindungsteil trägt (Fig. 1 - Fig. 2A).

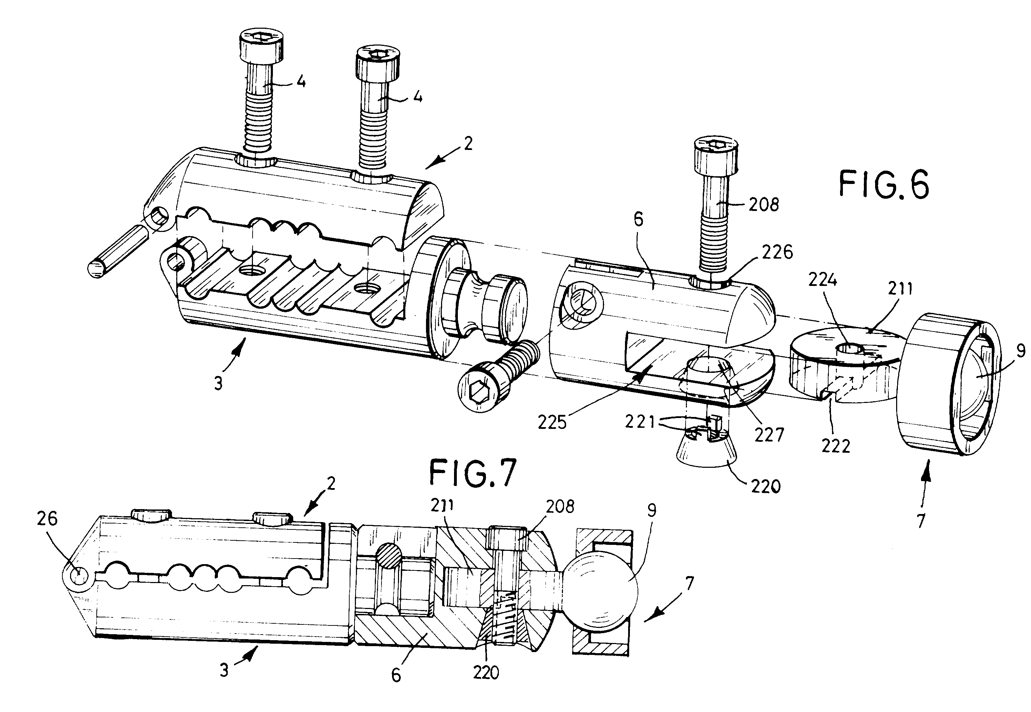

In einer zweiten Ausführungsform wird das angegebene Ziel durch eine axial kurze Verbindungseinheit mit einem Drehgelenk zwischen dem Muffen-Kugelgelenk und der Klemmkupplung erreicht (Fig. 6 und 7).

Mit diesen Ausführungen wird eine lösbare Klemmung mit einem Winkel von 90° und mehr zwischen der Längsachse der Knochenschraubenklemmung und der Längsachse des Fixateurkörpers ermöglicht.

Ausführungsformen der Erfindung werden anhand der Zeichnungen beschreiben, wobei
- Fig. 1: eine perspektivische Explosionszeichnung ist, z. T. im Längsschnitt, die eine Klemmkupplung der Erfindung darstellt,
- Fig. 2: ist eine ähnliche Ansicht, um die verbesserte Winkelstellung bei Verwendung der Kupplung gemäß Fig. 1 zu erläutern,
- Fig. 2A: ist ein Schnitt entlang der Linie 2A - 2A von Fig. 2,
- Fig. 3: ist eine Seitenansicht einer VerbindungsKupplung,
- Fig. 4: ist ein teilweiser Schnitt,
- Fig. 5: ist eine Ansicht auf eine weitere Ausführungsform einer Verbindung Kupplung,
- Fig. 6: ist in einer auseinandergezogenen Darstellungsweise eine weitere Ausführungsform einer gelenkigen Verbindung des Kugelgelenkanschlusses an die Klemmkupplung und
- Fig. 7: ist die Darstellung der Gegenstände gemäß Fig. 6 im eingesetzten Zustand.

Die Fig. 1 und 2 zeigen eine Klemmkupplung 1, die im wesentlichen aus zwei Halbschalen 2 und 3 besteht, die miteinander über Schrauben 4 befestigt werden können. Innerhalb der Halbschalen 2 und 3 sind Ausnehmungen 5 angeordnet und es ist zu verstehen, daß diese der Aufnahme und Fixierung von Knochenpins, Schrauben od dgl. dienen. Die Halbschale 3 hat ein vergrößertes oberes Ende, das dazu angepaßt ist, eine lösbare und sperrbare Drehbewegung eines Zapfens 6 einer Kugelgelenkverbindung 7 zu schaffen. Die Drehbewegung findet um die Längsachse 8 der Klemmkupplung 1 statt. Die Kugelgelenkverbindung 7 kann über Festschrauben befestigt werden, jedoch ist hier eine Bajonettverschlußverbindung für ein axiales Ende eines bekannten externen Fixateurs (nicht dargestellt) dargestellt. Die Kugelgelenkverbindung 7 besteht aus einer Kugel 9 und einem Ring 10, der für die abnehmbare Verbindung mit einem Fixateur dient.

Im weiteren wird die Kugelgelenkverbindung 7 als Muffen-Kugelverbindung benannt, bei der der Ring 10 gegenüber der beigeordneten Kugel 9 gehalten wird, die lose und drehbar zurückgehalten wird, bis sie von einer kompatiblen Einsteck-Kugelgelenkverbindung an einem der Enden eines Fixateurs beaufschlagt wird.
Ein Drehgelenk 11 ist zwischen den beiden Teilen 3 und 7 vorgesehen. Das Drehgelenk 11 hat einen Schwenkpin 12 und es ist zu verstehen, daß er ein Gewinde aufweist, durch das der Winkel des Drehgelenkes 11 durch die Verwendung eines Schraubschlüssels geklemmt bzw. fixiert werden kann. Die Kugel 9 ist mit dem Schwenkzapfen 12 über einen Schaftbereich 13 verbunden und die Halbschale 3 ist mit dem Schwenkzapfen 12 über eine Ösenformation an einem reduzierten Ende 14 eines Stiftbereiches 6 verbunden. Durch diese Anordnung wird es möglich, die Kugelgelenkverbindung 7 z. B. um 90° im Hinblick auf die Achse 8 der Klemmkupplung 1 zu bewegen und die Kugelgelenkverbindung in einer ausgewählten Winkelposition zu fixieren, so daß der Bereich der Verwendung der Klemmkupplung dadurch vergrößert wird.

Darüber hinaus wird eine größere Universalität der Winkeleinstellung des Drehgelenkes 11 durch die lösbare Drehbewegung des Zapfens 6 erreicht, der zur Drehung um die Achse 8 in dem vergrößerten oberen Ende 113 der Halbschale 3 fähig ist. Ein eingelassener Pin 16 in diesem oberen Ende 113 der Halbschale 8 beaufschlagt eine umlaufende Nut 15 in dem Zapfen 6, um den Zapfen 6 axial zurückzuhalten.

Ebenfalls ist das vergrößerte obere Ende 113 der Halbschale 3 bei 18 gespalten, um Klemmbacken zu schaffen, wobei ein Bolzen 17 mittels eines Schraubschlüssels betätigt werden kann, um eine ausgewählte Drehposition des Zapfens 6 (und daher ebenfalls der Drehachse des Pins 12) um die Längsachse 8 einzustellen.

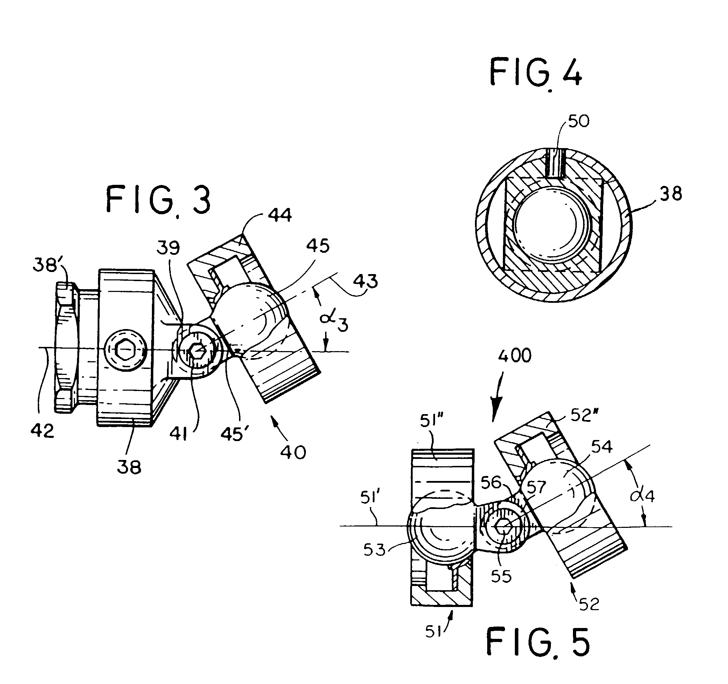

Die Ausführungsform gemäß Fig. 3 zeigt eine Verbindungskupplung 40, die eine drehbare Verbindung aufweist und die in Verbindung mit der Vorrichtung in den Fig. 1 und 2 verwendet werden, um einen noch größeren Bereich eines einstellbaren Klemmwinkels zu erhalten. Dies wird dadurch möglich, daß eine Drehverbindung über eine Schwenkachse 41 zwischen den Hälften 42 und 43 der Kupplung 40 erhalten wird und durch die Verwendung von Sperrmitteln für einen eingestellten Winkel der Drehverbindung. Das Sperrmittel für die Schwenkachse 41 ist so zu verstehen, daß es derjenigen ähnlich ist, die für den Zapfen in den Fig. 1 und 2 beschrieben worden ist. Genauer betrachtet umfaßt die linke Hälfte der Kupplung 40 ein Einsteckende 38 mit einem integrierten vorspringenden Bauteil 38', um selektiv das aufnehmende Kugelverbindungsmittel zu beaufschlagen und an dem anderen Ende der Achse 42 ist eine Ösenausbildung 39 für die Verbindung des Schwenkachse 41 mit einem flachen integrierten Schaftteil 45' der Kugel 45 ausgebildet.

In Fig. 3 ist ein Schwenkwinkel α₃ dargestellt und es ist zu verstehen, daß, wenn die Kupplung 40 mit der KnochenschraubenKlemmkupplung 1 und mit einem Fixateurkörperende verbunden und geklemmt wird, der maximale Winkel der einstellbaren Lage besonders groß sein kann. Obwohl die Schwenkachse 41 nur eine einzelne Querachse des Gelenkes zwischen seinem Einsteckende 38 und seinem Muffenende 44 mit der Kugel 45 bildet, wird der maximale Gesamtwinkel über den gesamten maximalen räumlichen Winkel ermöglicht im Hinblick auf die Drehfähigkeit jedes Ringes um seine zugeordnete Kugel 45. Das Einsteckende 38 ist über den Schlüsselpin 50, der auf das vorspringende Bauteil 38' wirkt, verriegelbar.

Fig. 5 stellt eine weitere Verbindungskupplung 400 dar, worin Muffen-Verbindungen 51, 52 mit ihren jeweiligen Kugelelementen 53, 54, eine lösbare geklemmte schwenkbare Verbindung ihrer Kugelelemente bei 55 und über Schaft- und Ösenausbildungen 56, 57 aufweisen. Daher schafft die integrierte Ausbildung der Öse 57 mit der Kugel 53 eine erste Mittelachse 51' der Kupplung 51 und ihrem Ring 51", und die integrierte Ausbildung eines flachen Schaftes 56 mit Kugel 54 schafft eine zweite Mittelachse 52' der Kupplung 52 und ihrem Ring 52", mit einem lösbaren geklemmten Gelenk dieser Achsen um die Schwenkachse bei 55 und damit den Winkel α₄.

Eine weitere Ausführungsform der Erfindung ist in den Fig. 6 und 7 dargestellt. Bei dieser Anordnung ist der mit der Klemmkupplung in Kontakt kommende Zapfen 6 als Gabel 225 ausgebildet, die nach vorne hin offen ist. In dem einen Schenkel der Gabel 225 ist eine konische Bohrung 227 vorgesehen, die zur Aufnahme einer entsprechend konisch ausgebildeten Nuß 220 dient, wobei diese Nuß 220 mit Innengewinde ausgerüstet ist. In dem anderen Schenkel ist eine Bohrung 226 vorgesehen, die zur Aufnahme einer Schraube 208 dient, wobei das Außengewinde der Schraube 208 mit dem Innengewinde der Nuß 220 zusammenarbeiten kann.

Die eigentliche Kugelgelenkverbindung 7 ist mit einer Befestigungsplatte 211 ausgerüstet, die eine Bohrung 224 aufweist und die weiterhin an ihrer Unterseite eine Nut 222 besitzt, die mit Vorsprüngen 221 an der Nuß dann in Kontakt kommen kann, wenn die Nuß in die zugehörige Bohrung 227 eingesetzt ist.

Im eingesetzten Zustand greift die Befestigungsplatte 211 zwischen die beiden Schenkel der Gabel 225 und die bohrung 224 der Befestigungsplatte 211 fluchtend mit den Bohrungen 226 und 227 in den Schenkeln der Gabel 225.

Die Befestigungsplatte 211 wird dann durch das Zusammenwirken der Schraube 208 mit der Nuß 220 festgelegt, kann aber gedreht werden.

Weiterhin ist erkennbar, daß auch der Zapfen 6 um seine Längsachse gedreht werden kann.

## Patentansprüche

1. Klemmkupplung (1) zur Festlegung von Knochenschrauben, Pins oder dergleichen und zum Verbinden derselben mit einem äußeren Fixateur, bestehend aus zwei mittels Schrauben (4) verbindbaren und Aufnahmen (5) für die Knochenschrauben, Pins oder dergleichen aufweisenden Halbschalen (2, 3) und einem an einer Halbschale mittels eines Zapfens (6) befestigten Kugelgelenkanschluß (7) mit einem Drehgelenk (11) zwischen der den Kugelgelenkanschluß (7) tragenden Halbschale (3) und dem Kugelgelenkanschluß (7), dadurch gekennzeichnet, daß der das Drehgelenk (11) tragende Zapfen (6) um seine Längsachse drehbar an einer der Halbschalen (3) anschließt

2. Vorrichtung nach Anspruch 1, dadurch gekennzeichnet, daß in dem in die Halbschale (3) eingreifenden Teil des Zapfens (6) eine umlaufende Nut (15) vorgesehen ist, in die ein in die Halbschale (3) einsetzbarer Stift (16) eingreift.

3. Vorrichtung nach Anspruch 1 oder 2, dadurch gekennzeichnet, daß die Halbschale (3) in ihrem den Zapfen (6) aufnehmenden oberen Ende (113) als Klemmbacke ausgebildet ist, deren Spalt (18) durch einen Schraubbolzen (17) beaufschlagbar ist.

4. Vorrichtung nach Anspruch 1 für die Fixation von Knochen mit einem äußeren Fixateur und Kugelgelenkanschlüssen, gekennzeichnet durch eine zwischen dem Kugelgelenkanschluß der Klemmkupplung (1) und dem Anschluß eines Fixateurs einstellbares Verbindungskupplung (40), die ein Muffenende (44) und ein Einsteckende (38) aufweist.

5. Vorrichtung nach Anspruch 4, dadurch gekennzeichnet, daß das Muffenende (44) der Verbindungskupplung (40) als Kugelgelenkverbindung und das Einsteckende (38') für eine Kugelgelenkverbindung ausgebildet ist.

6. Vorrichtung nach Anspruch 4 und 5, dadurch gekennzeichnet, daß das Einsteckende (38') um seine Längsachse drehbar und arretierbar ist.

7. Vorrichtung nach Anspruch 5 oder 6, dadurch gekennzeichnet, daß das Einsteckende (38') und das Muffenende (44) als Bajonettverschluß ausgebildet sind.

8. Vorrichtung nach einem der vorhergehenden Ansprüche, dadurch gekennzeichnet, daß die Verbindungskupplung (40 oder 400), bei welchem die Anschlußteile (38, 44 bzw. 51, 52) miteinander über eine Schwenkachse (41, 55) verbunden sind, die in jeder Winkelstellung der Anschlußteile arretierbar ist.

9. Vorrichtung nach Anspruch 8, dadurch gekennzeichnet, daß bei der Verbindungskupplung (40) die Anschlußteile (38', 44) einerseits als Kugelgelenkanschluß (44) und andererseits als Bajonettanschluß ausgebildet sind.

10. Vorrichtung nach Anspruch 8, dadurch gekennzeichnet, daß bei der Verbindungskupplung (400) die Anschlußteile einerseits als Kugelgelenkanschluß (52) und andererseits ebenfalls als Kugelgelenkanschluß (51) ausgebildet sind.

11. Vorrichtung nach einem der Ansprüche 1 bis 3, gekennzeichnet durch eine konische Nuß (220) mit Innengewinde, eine gabelförmige Ausbildung des Zapfens (6) (Gabel 225), eine konische Bohrung (227) zur Aufnahme der Nuß (220) in einem Schenkel des Zapfens (6) und eine Bohrung (226) zur Aufnahme einer Schraube (208) mit Außengewinde im anderen Schenkel der Gabel (225) und eine Befestigungsplatte (211) mit zentraler Bohrung (224), die die Kugelgelenkverbindung (7) trägt, wobei im eingesetzten Zustand der Befestigungsplatte (211) in der Gabel (225) die mit Innengewinde versehene Bohrung der Nuß (220), die Bohrung (227) in dem einen Schenkel, die Bohrung (226) in dem anderen Schenkel und die Bohrung (224) in der Befestigungsplatte (211) miteinander fluchten.

12. Vorrichtung nach Anspruch 11, dadurch gekennzeichnet, daß an der mit der Nuß (220) in Kontakt kommenden Seite der Befestigungsplatte eine mit Vorsprüngen (221) an der Nuß (220) zusammenwirkende Nut (222) vorgesehen ist.

## Claims

1. Clamping coupling (1) for the fixing of bone screws, pins or the like and for connecting these to an external fixateur, the said clamping coupling consisting of two half-shells (2, 3) connectable by means of screws (4) and having receptacles (5) for the bone screws, pins or the like and of a ball-joint connection (7) fastened to one half-shell by means of a journal (6) and having a rotary joint (11) between the half-shell (3) carrying the ball-joint connection (7) and the ball-joint connection (7), characterized in that the journal (6) carrying the rotary joint (11) is connected rotatably about its longitudinal axis to one of the half-shells (3).

2. Device according to Claim 1, characterized in that there is provided in that part of the journal (6) which engages into the half-shell (3) a peripheral groove (15) into which engages a peg (16) capable of being inserted into the half-shell (3).

3. Device according to Claim 1 or 2, characterized in that the half-shell (3) is designed, in its upper end (113) receiving the journal (6), as a clamping jaw, the gap (18) of which is capable of being acted upon by a screw bolt (17).

4. Device according to Claim 1 for the fixing of bones to an external fixateur and ball-joint connections, characterized by a connecting coupling (40) which is capable of being introduced between the ball-joint connection of the clamping coupling (1) and the connection of a fixateur and which has a socket end (44) and a plug-in end (38).

5. Device according to Claim 4, characterized in that the socket end (44) of the connecting coupling (40) is designed as a ball-joint connection and the plug-in end (38') is designed for a ball-joint connection.

6. Device according to Claims 4 and 5, characterized in that the plug-in end (38') is rotatable about its longitudinal axis and is lockable.

7. Device according to Claim 5 or 6, characterized in that the plug-in end (38') and the socket end (44) are designed as a bayonet fastening.

8. Device according to one of the preceding claims, characterized in that, in the connecting coupling (40 or 400), the connection parts (38, 44 and 51, 52) are connected to one another via a pivot shaft (41, 55) which is lockable in any angular position of the connection parts.

9. Device according to Claim 8, characterized in that, in the connecting coupling (40), the connection parts (38', 44) are designed, on the one hand, as a ball-joint connection (44) and, on the other hand, as a bayonet connection.

10. Device according to Claim 8, characterized in that, in the connecting coupling (400), the connection parts are designed, on the one hand, as a ball-joint connection (52) and, on the other hand, likewise as a ball-joint connection (51).

11. Device according to one of Claims 1 to 3, characterized by an internally threaded conical plug (220), a fork-shaped design of the journal (6) (fork 225), a conical bore (227) for receiving the plug (220) in one leg of the journal (6), a bore (226) for receiving an externally threaded screw (208) in the other leg of the fork (225), and a fastening plate (211) having a central bore (224) and carrying the ball-joint connection (7), in the state in which the fastening plate (211) is inserted in the fork (225) the internally threaded bore of the plug (220), the bore (227) in one leg, the bore (226) in the other leg and the bore (224) in the fastening plate (211) being in alignment with one another.

12. Device according to Claim 11, characterized in that a groove (222) co-operating with projections (221) on the plug (220) is provided on that side of the fastening plate which comes into contact with the plug (220).

## Revendications

1. Système d'accouplement à serrage (1) destiné à la fixation de vis pour fractures osseuses, broches ou dispositifs similaires, et à la liaison de ceux-ci avec un fixateur externe, composé de deux demi-coquilles (2, 3) pouvant être reliées par deux vis (4) et présentant des logements (5) pour les vis de fracture, broches ou dispositifs similaires, et d'une articulation à rotule (7) fixée à une demi-coquille au moyen d'un pivot (6) et dotée d'une liaison articulée (11), entre l'articulation à rotule (7) et la demi-coquille (3) portant l'articulation (7), *caractérisé en ce que* le pivot (6) portant la liaison articulée (11) et tournant autour de son axe longitudinal, est relié à une des demi-coquilles (3).

2. Système selon la revendication 1, caractérisé en ce qu'il est prévu dans la partie du pivot (6) qui pénètre dans la demi-coquille (3), une gorge périphérique (15) dans laquelle pénètre un goujon (16) pouvant être inséré dans la demi-coquille (3).

3. Système selon les revendications 1 ou 2, caractérisé en ce que la demi-coquille (3) a la forme d'une mâchoire de serrage au niveau de son extrémité supérieure (113) recevant le pivot (6), dont la fente (18) peut être sollicitée par un boulon fileté (17).

4. Système selon la revendication 1, destiné à la fixation d'os à un fixateur externe et des raccords articulés à rotule, caractérisé en ce qu'il comprend un accouplement de liaison (40) réglable entre l'articulation à rotule de l'accouplement à serrage (1) et le raccord d'un fixateur, accouplement présentant une extrémité femelle (44) et une extrémité mâle (38).

5. Système selon la revendication 4, caractérisé en ce que l'extrémité femelle (44) de l'accouplement de liaison (40) a la forme d'une articulation à rotule et que l'extrémité mâle (38') est conçue pour une articulation à rotule.

6. Système selon la revendication 4 ou 5, caractérisé en ce que l'extrémité mâle (38') est articulée autour de son axe longitudinal et peut être bloquée.

7. Système selon la revendication 5 ou 6, caractérisé en ce que l'extrémité mâle (38') et l'extrémité femelle (44) ont la forme d'un joint à baïonnette.

8. Système selon l'une des revendications précédentes, caractérisé en ce que l'accouplement de liaison (40 ou 400), sur lequel les éléments de raccordement (38, 44 ou 51, 52) sont reliés l'un à l'autre par un axe de pivotement (41, 55), peut être bloqué dans n'importe quelle position angulaire des éléments de raccordement.

9. Système selon la revendication 8, caractérisé en ce que, sur l'accouplement de liaison (40), les éléments de raccordement (38', 44) ont d'un côté la forme d'une articulation à rotule (44) et de l'autre la forme d'un joint à baïonnette.

10. Système selon la revendication 8, caractérisé en ce que, sur l'accouplement de liaison (400), les éléments de raccordement ont d'un côté la forme d'une articulation à rotule (52) et de l'autre également la forme d'une articulation à rotule (51).

11. Système selon l'une des revendications 1 à 3, caractérisé en ce qu'il comporte une noix conique (220) à pas de vis intérieur, une forme en fourche du pivot (6) (fourche 225), un perçage conique (227) destiné à recevoir la noix (220) dans une branche du pivot (6) et un perçage (226) destiné à recevoir une vis (208) à pas de vis extérieur dans l'autre branche de la fourche (225) et une plaque de fixation (211) à perçage central (224) qui porte la liaison articulée à rotule (7), sachant que, lorsque la plaque de fixation (211) est en position d'insertion dans la fourche (225), le perçage à pas de vis intérieur de la noix (220), le perçage (227) d'une branche, le perçage (226) de l'autre branche et le perçage (224) de la plaque de fixation (211) sont alignés les uns avec les autres.

12. Système selon la revendication 11, caractérisé en ce que, du côté de la plaque de fixation qui vient en contact avec la noix (220), il est prévu une gorge (222) qui interagit avec des ergots (221) situés sur la noix (220).
